(19) Europäisches Patentamt / European Patent Office / Office européen des brevets

(11) **EP 4 553 496 B1**

(12) **FASCICULE DE BREVET EUROPÉEN**

(45) Date de publication et mention
de la délivrance du brevet:
**08.04.2026 Bulletin 2026/15**

(21) Numéro de dépôt: **24211783.6**

(22) Date de dépôt: **08.11.2024**

(51) Classification Internationale des Brevets (IPC):
***G01N 31/22*** *(2006.01)* ***B01J 20/28*** *(2006.01)*

(52) Classification Coopérative des Brevets (CPC):
**G01N 31/223; B01J 20/28083**

(54) **PROCÉDÉ DE DÉTECTION DE COMPOSÉS DE TYPE AMINE DANS L'AIR**

VERFAHREN ZUM NACHWEIS VON AMINVERBINDUNGEN IN DER LUFT

METHOD FOR DETECTING AMINE COMPOUNDS IN AIR

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **09.11.2023 FR 2312243**

(43) Date de publication de la demande:
**14.05.2025 Bulletin 2025/20**

(73) Titulaires:
• **Commissariat à l'Energie Atomique et aux Energies Alternatives**
**75015 Paris (FR)**
• **Centre National de la Recherche Scientifique**
**75016 Paris (FR)**

(72) Inventeurs:
• **TRAN THI, Thu-Hoa**
**92120 MONTROUGE (FR)**
• **NGUYEN, Trung Hieu**
**75005 PARIS (FR)**
• **LE CHEVALLIER, Guillaume**
**91191 GIF SUR YVETTE CEDEX (FR)**

(74) Mandataire: **Plasseraud IP**
**104 Rue de Richelieu**
**CS92104**
**75080 Paris Cedex 02 (FR)**

(56) Documents cités:
US-A- 5 719 061  US-A1- 2012 113 421
US-A1- 2012 295 363  US-A1- 2020 230 542

**Description**

**Domaine technique**

La présente invention concerne un nouveau procédé de détection et optionnellement de quantification de composés de type amine, en particulier de l'hydrazine (N2H4), dans l'air.

L'hydrazine est une substance classée CMR (CIRC 2B-UE 1B). Elle a intégré en 2011 la liste des substances candidates extrêmement préoccupantes (en anglais « candidate list of substances of very high concern ») du règlement REACH. En 2017, l'Union Européenne a abaissé d'un facteur 10 sa VLEP (Valeur Limite d'Exposition Professionnelle sur 8 H) imposant un nouveau seuil de 10 ppb (0,013 mg/m3), applicable au plus tard le 17 janvier 2020. Outre le volet sanitaire humain, l'hydrazine est classée très toxique pour les organismes aquatiques (classement UE H400 et H410).

Du fait de son caractère CMR, l'hydrazine n'est plus un comburant pour les fusées, mais reste utilisée dans de nombreux domaines, notamment comme intermédiaire de synthèse organique dans les industries pharmaceutiques et chimiques, ou agent gonflant pour les mousses de polymères, ou comme réducteur de sels de métaux ou encore en tant qu'inhibiteur de corrosion dans l'eau de circuits des chaudières industrielles, par exemple.

Du fait de sa toxicité, l'évaluation des risques associés aux rejets d'hydrazine environnementaux est importante et nécessite de pouvoir détecter et optionnellement quantifier la présence d'hydrazine dans l'environnement.

L'évaluation de ces risques est à ce jour fortement limitée par la métrologie associée à cette substance, en particulier concernant la mesure directe in-situ. En effet, malgré le fait qu'il existe des méthodes analytiques relativement sensibles et spécifiques (lecture finale par HPLC-UV), celles-ci ne peuvent pas être mises en œuvre facilement ni être compatibles avec des pratiques industrielles. Les méthodes analytiques « à réponse instantanée » ne sont ni assez sélectives ni assez précises et sensibles pour permettre d'évaluer les concentrations d'hydrazine gazeux attendues dans les rejets ultimes.

La détection et la quantification d'hydrazine sont actuellement réalisées via une méthode indirecte. Il s'agit dans cette méthode de prélever l'air ambiant à analyser et de piéger l'hydrazine dans une cartouche acidifiée, puis de la désorber et de la faire réagir avec une solution de réactif. Les composés d'addition formés sont ensuite séparés par chromatographie en phase liquide et analysés optiquement.

Outre la durée de cette méthode qui implique plusieurs étapes difficilement réalisables directement sur le lieu du prélèvement, les interférences potentielles avec d'autres composés gazeux azotés ne sont pas connues notamment en présence potentielle d'interférents servant à alcaliniser l'eau ou issus de la dégradation de l'hydrazine, tels que l'éthanolamine (NH2EtOH), la morpholine, ou l'ammoniac (NH3).Il est important de pouvoir mesurer sélectivement l'hydrazine de manière directe dans une gamme de concentration de 1 à 100 ppb dans de l'air sec à très humide et en présence d'interférents dont la concentration peut être 50 à 100 fois plus élevée que celle de l'hydrazine.

Le procédé objet de la présente invention vient apporter une solution à ces problématiques.

En effet, le procédé selon l'invention permet de détecter et optionnellement de quantifier sélectivement l'hydrazine en présence de vapeur d'eau, de composés organiques volatils et d'autres composés azotés tels que l'éthanolamine ($NH_2EtOH$), la morpholine ou l'ammoniac. Au contact de l'hydrazine, le capteur utilisé change de couleur et l'intensité de coloration est proportionnelle à la teneur d'hydrazine, ce qui permet de détecter et potentiellement quantifier ce composé.

**Technique antérieure**

La détection de l'hydrazine a fait l'objet de nombreux travaux et les méthodes de détection sont aussi nombreuses que variées en raison de la forte réactivité chimique de ce composé. De ce fait, sont décrites ici uniquement les méthodes proposées dans la littérature, aptes à couvrir la gamme de concentration recherchée soit de 1,3 à 130 $\mu$g.m-3 (soit de 1 à 100 ppb).

Les méthodes de mesure de l'hydrazine dans l'air sont moins nombreuses que celles pour la phase liquide, notamment dans la gamme ciblée de 1,3 à 130$\mu$g.m-3 (soit de 1 à 100 ppb. La méthode actuelle de mesure est décrite dans la Fiche 21 de l'INRS. Elle repose sur l'utilisation du benzaldéhyde [1]. L'hydrazine est prélevée par aspiration d'air au travers d'un tube rempli d'un adsorbant inerte (Chromosorb P NAW ou équivalent) de granulométrie 3060 Mesh, imprégné d'acide sulfurique. Le contenu de la cartouche est désorbé à l'eau déionisée et une dérivation par le benzaldéhyde est réalisée. Le composé d'addition, la benzalazine, est mesuré en chromatographie en phase liquide (HPLC) couplée à une détection optique dans l'UV [2, 3]. Cette méthode permet de détecter 30 ppb d'hydrazine en 15 minutes de prélèvement.

Une variante de cette méthode est l'utilisation de cassette contenant deux filtres en fibre de verre imprégnés d'acide sulfurique. L'extraction de l'hydrazine est réalisée avec une solution tampon d'EDTA et la dérivation est réalisée avec le benzaldéhyde. La benzalazine formée est mesurée en chromatographie en phase liquide couplée à une détection optique dans l'UV [4]. Cette méthode de dosage s'avère sensible, puisqu'il est possible de détecter 0,017 ppb de $N_2H_4$.

Ces deux méthodes ne permettent pas une mesure directe sur le lieu du prélèvement et l'interférence d'ammoniac ou d'autres amines à de fortes concentrations 50 à 100 fois supérieures à celle de l'hydrazine n'est pas connue.

On trouve également des méthodes de mesure directe. Dans le cadre de la surveillance de l'exposition des travailleurs à l'hydrazine, au monométhylhydrazine (MMH) et au 1,1-dimethylhydrazine (UDMH) utilisés en tant que carburants pour les fusées dans les bases aériennes et dans les centres spatiaux aux États-Unis, plusieurs dosimètres colorimétriques ont été développés et commercialisés par des laboratoires [5, 6]. Le principe est basé sur l'incorporation d'un aldéhyde aromatique comme la vanilline, le para-diméthylaminobenzaldéhyde (pDMAB) ou le 2,4-Dinitrobenzaldéhyde sur un papier filtre ou une surface inerte. L'hydrazine et le MMH réagissent avec la vanilline ou avec le 2,4-Dinitrobenzaldéhyde pour former un composé de couleur jaune, tandis que le produit formé avec le pDMAB est orange. L'UDMH réagit avec le 2,4-Dinitrobenzaldéhyde pour former un composé de couleur jaune et il n'y a pas de réaction avec la vanilline et le pDMAB. Les dosimètres colorimétriques à base de la vanilline et du para-diméthylaminobenzaldéhyde sont commercialisés par la société DODTEC [7] et CHEMSEE [8]. Ils ne permettent pas une quantification exacte mais uniquement d'estimer les teneurs de l'hydrazine, du monométhylhydrazine dans l'air à partir de 25 ppb et jusqu'à 1,2 ppm.

Dans le brevet US005719061A [9], Rose-Pehrsson et al. proposent un procédé permettant la détection et quantification sélective d'hydrazine, de monométhylhydrazine et de 1,1-diméthylhydrazine liquide ou gazeuse par dérivation avec des carboxaldéhydes aromatiques et analyse fluorimétrique. La sélectivité est basée sur la réactivité de 3 agents de dérivation, l'ortho-phthalaldéhyde (OPA), le napthalène 2,3- dicarboxaldéhyde (NDA) et l'anthracène 2,3-dicarboxaldéhyde (ADA) avec l'hydrazine, la monométhylhydrazine et de 1,1-diméthylhydrazine en fonction du pH de la solution de réactif. Ces auteurs ont donc mis au point un dispositif complexe qui permet de pomper l'air ambiant pour le faire barboter dans une solution de réactif dont la composition (OPA ou NDA ou ADA) et le pH doivent être modifiés pour l'obtention de la sélectivité. Même si la limite de détection atteinte est le ppb, l'analyse du mélange gazeux à analyser nécessite de nombreuses étapes de changement de réactifs et de pH, suivies d'analyses fluorimétriques. Par ailleurs, il n'y a pas d'étude d'interférence, notamment avec d'autres amines à des concentrations 50 à 100 fois plus fortes, susceptibles de modifier le pH de la solution. US2012295363A1 divulgue un capteur chimique solide basé sur un matériau sol-gel poreux dopé avec des molécules sondes 4-(diméthylamino)-cinnamaldéhyde DMABA. Ce capteur permet la détection et la quantification de composés chimiques tels que indole et ses dérivés, mais aussi des amines tels que hydrazine et urobilinogène.

Pour des mesures précises, quelques appareils commerciaux existent. Le détecteur électrochimique portable de la société Interscan, modèle 4180-100b [10] permet de détecter l'hydrazine dans la plage de 10 à 100 ppb en moins de 1 seconde, avec une limite de détection élevée de 10 ppb. La méthode n'est en outre pas sélective car le capteur détecte également NH3, NOx, CO et les autres amines organiques.

Une grande sensibilité peut être obtenue à l'aide d'appareils équipés d'un détecteur à photoionisation (PID) comme ppbRAE 3000 de la société RAE [11]. Le détecteur à photoionisation, équipé d'une lampe de 10.6 eV, permet d'ioniser l'hydrazine et de mesurer quelques ppbs en 3 secondes. La détection n'est cependant pas sélective car un grand nombre de composés organiques volatils présents dans l'air dont le potentiel d'ionisation est inférieur à 10,6 eV sont également détectés, comme NH3, l'éthanolamine et la morpholine.

L'ionisation de l'hydrazine suivie d'une mesure de mobilité des ions à l'aide de la spectrométrie à mobilité ionique, en anglais Ion Mobility Spectrometer (IMS) permet d'atteindre des teneurs de l'ordre de la dizaine de ppb (20 - 30 ppb) tout en étant sélectif avec le choix du gaz porteur. Avec l'utilisation d'une source radioactive, l'IMS (comme le détecteur portable SABRE 4000 [12] ou ChemPro100i d'Environics [13] doit être sous la responsabilité d'une personne compétente en radioprotection. Cette technique est privilégiée par les armées et polices pour la détection des armes chimiques et des produits illicites. Son application dans le domaine public s'est développée plus récemment avec le développement de nouvelles sources d'ionisation non-radioactives (effet Corona) comme le détecteur portable PAIMS de la société MaSaTECH [14] ou LCD 3.3 de Smiths Detection [15].

L'état de l'art des mesures d'hydrazine montre que la seule méthode simple actuellement adoptée, utilisant comme réactif le benzaldéhyde, peut être utilisée avec une bonne sélectivité et sensibilité. Cependant, la mesure de l'hydrazine dans le compartiment air nécessite les étapes d'adsorption puis de désorption et de dérivation suivies de l'analyse optique qui sont difficilement réalisables sur site. De plus, pour cette méthode, les interférences avec de fortes concentrations de NH3, d'éthanolamine ou de morpholine gazeux dans l'atmosphère ne sont pas connues.

Il existe donc un réel besoin d'un procédé de détection directe et optionnellement de quantification directe de l'hydrazine dans l'air, sélectif de l'hydrazine, compatible avec la présence de fortes concentrations d'interférents, facile à mettre en œuvre sur le terrain.

Le procédé selon l'invention vient répondre à ces problématiques.

**Résumé**

Un premier objet de l'invention est un capteur nanoporeux composé d'une matrice sol-gel silicatée nanoporeuse renfermant une composition de réactifs, ladite composition de réactifs comprenant un mélange de 4-(diméthylamino)-cinnamaldéhyde et d'acide polystyrène sulfonique .

Un autre objet de l'invention est un procédé de préparation d'un capteur nanoporeux selon l'invention, ledit procédé comprenant les étapes suivantes :

a. Synthèse d'un sol à partir d'un précurseur organosilylé, la synthèse étant effectuée dans un solvant, ledit solvant comprenant de l'eau, en présence de 4-(diméthylamino)-cinnamaldéhyde et d'acide polystyrène sulfonique ;

b. Gélification du sol obtenu à l'étape a), de façon à obtenir un gel ;

c. Séchage du gel obtenu à l'étape b), de façon à obtenir un capteur nanoporeux.

La présente invention a également pour objet un procédé de détection d'au moins un composé de type amine, ledit composé étant choisi parmi l'hydrazine, l'éthanolamine, l'ammoniac et la morpholine, à l'aide d'un capteur nanoporeux selon l'invention, ledit procédé comprenant les étapes de :

a. mise en contact d'un échantillon gazeux à analyser avec ledit capteur nanoporeux,
b. détection du ou des dit(s) composé(s) de type amine sur ledit capteur nanoporeux.

Un autre objet de l'invention est l'utilisation d'un capteur nanoporeux selon l'invention, pour la détection et/ou la quantification d'au moins un composé de type amine, ledit composé étant choisi parmi l'hydrazine, l'éthanolamine, l'ammoniac et la morpholine.

La présente invention concerne également un dispositif de détection d'au moins un composé de type amine, ledit composé étant choisi parmi l'hydrazine, l'éthanolamine, l'ammoniac et la morpholine, dans un échantillon gazeux à analyser, ledit dispositif comprenant une cellule renfermant un capteur nanoporeux selon l'invention et comprenant :

- une entrée de gaz ;

- une sortie de gaz ;

- une entrée optique ;

- une sortie optique.

## Brève description des dessins

D'autres caractéristiques, détails et avantages apparaîtront à la lecture de la description détaillée ci-après, et à l'analyse des dessins annexés, sur lesquels :

**Fig. 1**

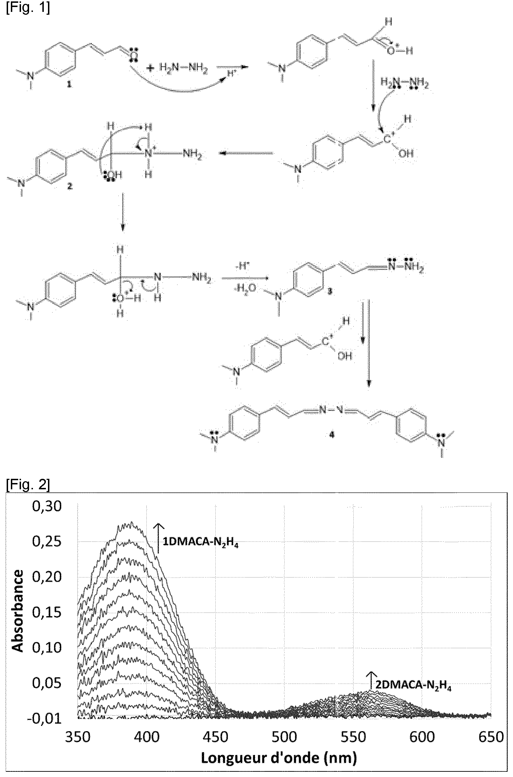

[Fig. 1] montre le schéma de la réaction entre $N_2H_4$ et DMACA catalysée en milieu acide avec formation de DMACA-$N_2H_4$ (composé 3) et 2DMACA-$N_2H_4$ (composé 4).

**Fig. 2**
[Fig. 2] montre les spectres différentiels obtenus à différents temps montrant l'évolution de l'absorbance des complexes DMACA-$N_2H_4$ et 2DMACA-$N_2H_4$ dans le capteur nanoporeux Hy3 au cours de l'exposition du capteur à un flux de mélange gazeux contenant 14 ppb de $N_2H_4$. Un spectre est collecté toutes les 5 min de 0 à 60 min.

**Fig. 3**

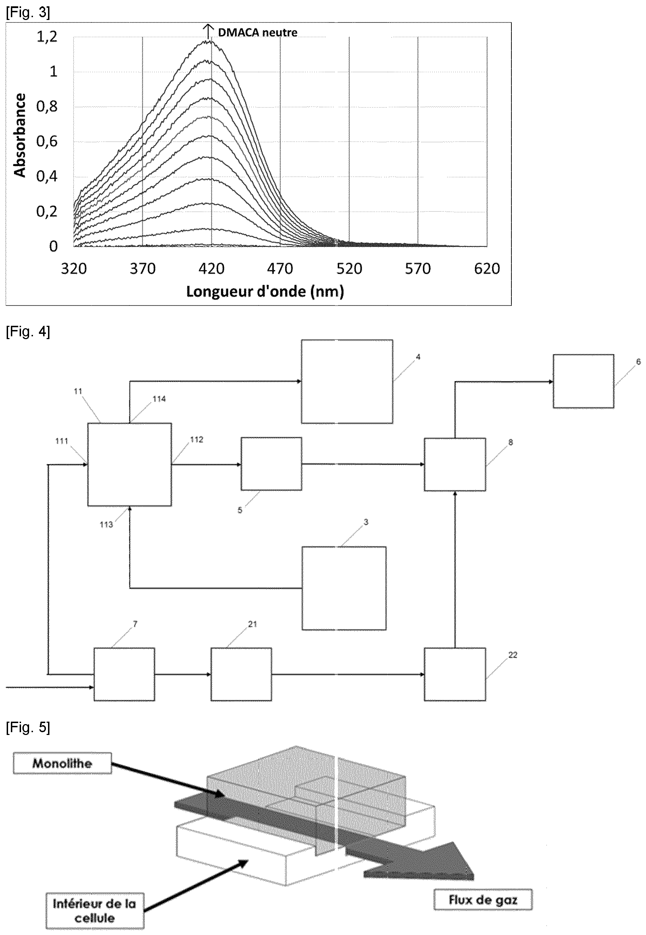

[Fig. 3] montre les spectres différentiels obtenus à différents temps montrant l'évolution de l'absorbance du DMACA neutre dans le capteur nanoporeux Hy1 au cours de l'exposition du capteur à un flux de mélange gazeux contenant 5 ppm de $NH_3$. Un spectre est collecté toutes les 5 min de 0 à 50 min.

**Fig. 4**
[Fig. 4] montre le schéma d'un exemple de dispositif selon l'invention.

**Fig. 5**
[Fig. 5] montre le schéma d'un exemple de circulation du flux de gaz autour du capteur nanoporeux placé dans la cellule selon un mode de réalisation particulier de l'invention.

**Fig. 6**

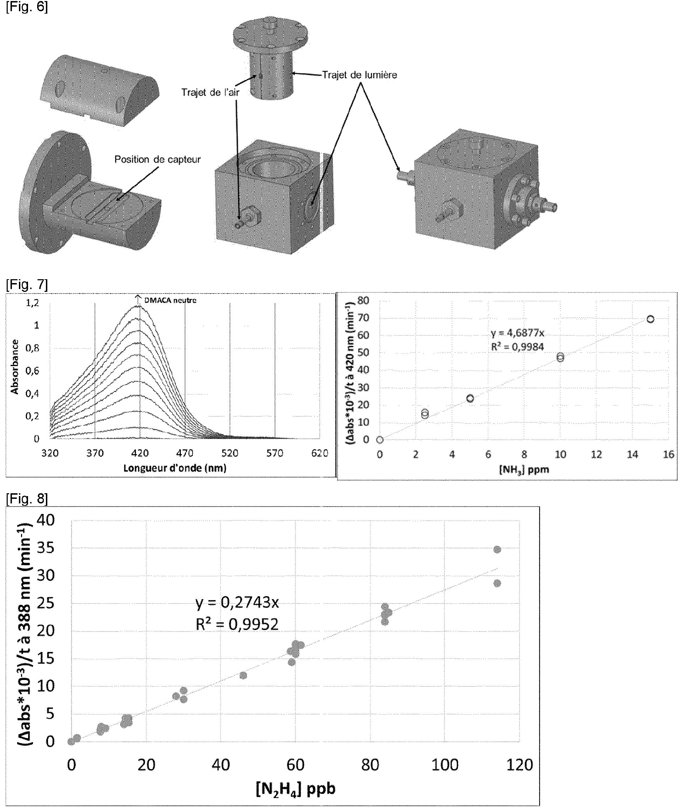

[Fig. 6] montre le schéma d'un exemple de cellule du dispositif selon l'invention.

**Fig. 7**

[Fig. 7] montre : à gauche l'évolution du spectre d'absorption du capteur Hy1 exposé à 5 ppm de $NH_3$, un spectre est collecté toutes les 5 minutes pendant 50 minutes ; à droite la variation de la vitesse de formation de DMACA neutre en fonction de la concentration de $NH_3$. Flux = 200 ml·min$^{-1}$, %HR = 50%.

**Fig. 8**

[Fig. 8] montre la courbe d'étalonnage du capteur Hy3 pour la détection de $N_2H_4$. Variation de la vitesse de formation du complexe 1DMACA-$N_2H_4$ à 388 nm en fonction de la concentration de $N_2H_4$. Flux = 200 mL·min$^{-1}$, %HR = 50%.

**Fig. 9**

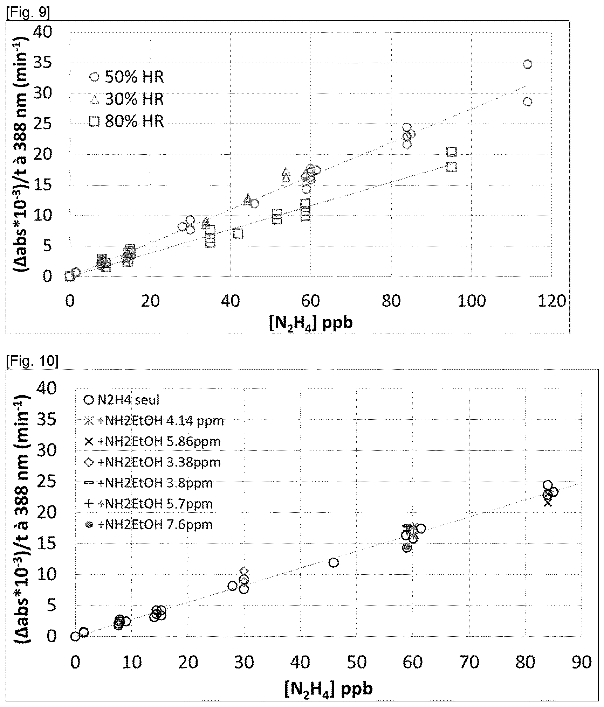

[Fig. 9] montre l'effet de l'humidité relative du mélange gazeux sur la vitesse de formation du complexe 1DMACA-$N_2H_4$ en fonction de la concentration de $N_2H_4$. Flux = 200 mL·min$^{-1}$.

**Fig. 10**

[Fig. 10] montre l'effet de la présence d'un interférent potentiel, $NH_2EtOH$, sur la réponse du capteur Hy3 à l'hydrazine. Flux = 200 mL·min$^{-1}$, HR = 50%.

**Fig. 11**

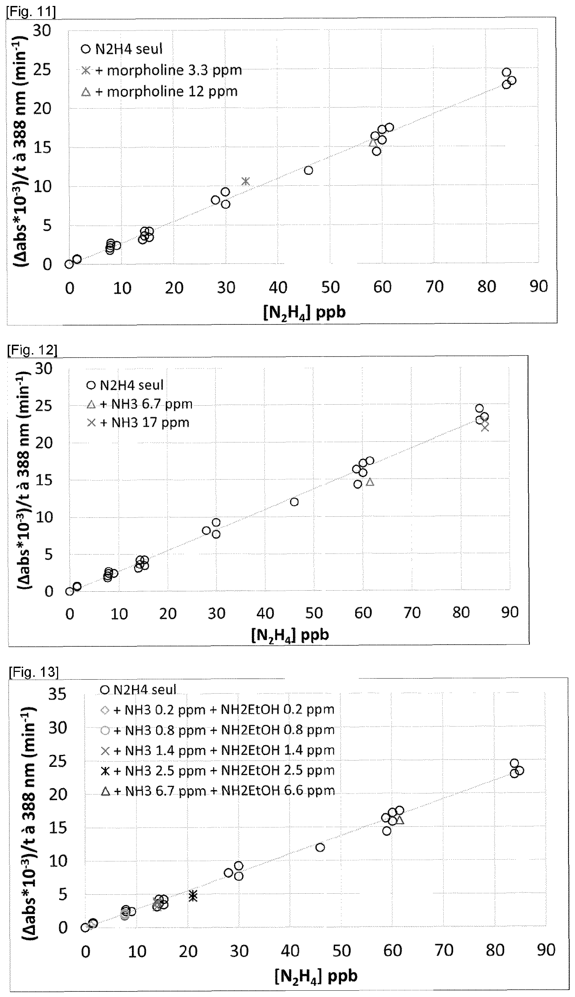

[Fig. 11] montre l'effet de la présence d'un interférent potentiel, morpholine, sur la réponse du capteur Hy3 à l'hydrazine. Flux = 200 mL·min$^{-1}$, HR = 50%.

**Fig. 12**

[Fig. 12] montre l'effet de la présence d'un interférent potentiel, $NH_3$, sur la réponse du capteur Hy3 à l'hydrazine. Flux = 200 mL·min$^{-1}$, HR = 50%.

**Fig. 13**

[Fig. 13] montre l'effet de la présence de deux interférents potentiels, $NH_3$ et $NH_2EtOH$, sur la réponse du capteur Hy3 à l'hydrazine. Flux = 200 mL·min$^{-1}$, HR = 50%.

**Fig. 14**

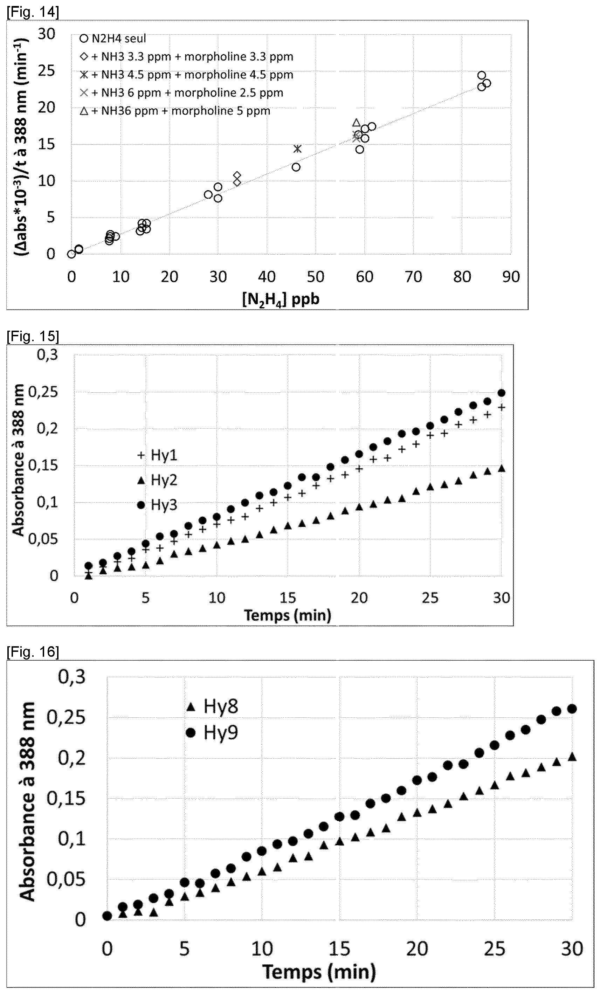

[Fig. 14] montre l'effet de la présence de deux interférents potentiels, $NH_3$ et morpholine, sur la réponse du capteur Hy3 à l'hydrazine. Flux = 200 mL·min$^{-1}$, HR = 50%.

**Fig. 15**

[Fig. 15] montre la comparaison des réponses des capteurs Hy1, Hy2 et Hy3 à 30 ppb de $N_2H_4$. Flux = 200 mL·min$^{-1}$, % HR = 50%.

**Fig. 16**

[Fig. 16] montre la comparaison des réponses des capteurs Hy8 et Hy9 à 40 ppb de $N_2H_4$. Flux = 200 mL·min$^{-1}$, %HR = 50%.

**Fig. 17**

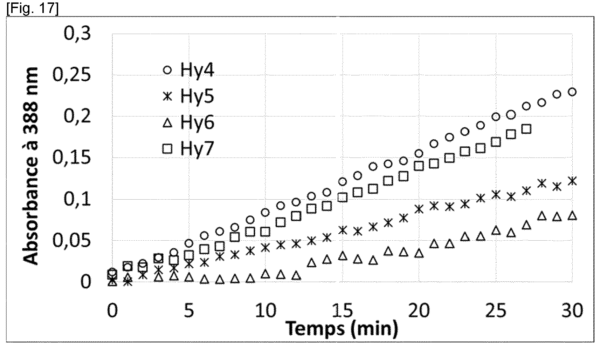

[Fig. 17] montre la comparaison des réponses des capteurs Hy4, Hy5, Hy6 et Hy7 à 25 ppb de $N_2H_4$. Flux = 200 mL·min$^{-1}$, %HR = 50%.

## Description détaillée

La présente invention a pour objet un capteur nanoporeux composé d'une matrice sol-gel silicatée nanoporeuse renfermant une composition de réactifs, ladite composition de réactifs comprenant un mélange de 4-(diméthylamino)-cinnamaldéhyde et d'acide polystyrène sulfonique.

Au sens de la présente invention, on entend par matrice sol-gel silicatée un matériau obtenu par un procédé sol-gel consistant à utiliser comme précurseurs des alcoxydes de silicium de formule Si(OR)x où R est un groupement alkyle.

Lors du procédé sol-gel, les groupements alkoxy (OR) sont hydrolysés en présence d'eau en groupements silanols (Si-OH). Ces derniers se condensent en formant des liaisons siloxane (Si-O-Si-). Il se forme alors des petites particules de taille généralement inférieure à 1 $\mu$m, qui s'agrègent et forment des amas qui restent en suspension sans précipiter, formant un sol. L'augmentation des amas et leur condensation augmentent la viscosité du milieu qui gélifie. Un matériau solide poreux est obtenu par séchage du gel, avec l'expulsion du solvant en dehors du réseau polymérique formé (synérèse). Les matrices sol-gel obtenues à partir d'alcoxydes de silicium de formule $Si(OR)_x$ sont appelés matrices sol-gel silicatées dans la présente demande.

Au sens de la présente invention, le terme nanoporeux signifie : dont les pores présentent une taille inférieure à 100 nm.

Selon un mode de réalisation particulier, la matrice nanoporeuse selon l'invention est une matrice essentiellement microporeuse. Ainsi, selon ce mode de réalisation, le capteur peut aussi être décrit comme essentiellement microporeux. On entend par matériau essentiellement microporeux (capteur essentiellement microporeux ou matrice essentiellement microporeuse) un matériau dont au moins 80% des pores sont des micropores.

Les micropores sont caractérisés selon la définition IUPAC par une largeur qui n'excède pas 2 nm (IUPAC. Compendium of Chemical Terminology, 2nd ed. (the "Gold Book"). Compiled by A. D. McNaught and A. Wilkinson. Blackwell Scientific Publications, Oxford (1997). Version en ligne (2019-) créée par S. J. Chalk. ISBN 0-9678550-9-8. https://doi.org/10.1351/goldbook).

Au contact d'un composé de type amine, et notamment de l'hydrazine gazeuse, le capteur nanoporeux selon l'invention change de couleur du fait de la formation des complexes DMACA-$N_2H_4$ et 2DMACA-$N_2H_4$ par réaction entre l'hydrazine ($N_2H_4$) et le 4-(diméthylamino)-cinnamaldéhyde (DMACA) en présence d'acide polystyrène sulfonique qui permet de catalyser cette réaction. Les variations d'absorbance sont corrélées à la teneur d'hydrazine dans l'air.

La réaction entre l'hydrazine ($N_2H_4$) et le 4-(diméthylamino)-cinnamaldéhyde (DMACA) est avantageusement catalysée par un acide dérivé d'un polymère, en particulier l'acide polystyrène sulfonique. Le choix de ce type d'acide et en particulier de l'acide polystyrène sulfonique a de nombreux avantages.

Tout d'abord, cet acide est un polymère de haut poids moléculaire qui comporte une fonction acide $SO_3H$ pour chaque monomère styrénique. De ce fait, le nombre de protons disponibles est particulièrement élevé et permet d'ajuster l'acidité des pores pour catalyser la réaction entre le DMACA et $N_2H_4$.

Par ailleurs, les acides inorganiques et les acides organiques de petit poids moléculaire présentent l'inconvénient de possibles dégagements de vapeur d'acide lors de l'étape de séchage de la matrice sol-gel, l'acide étant donc susceptible de s'évaporer également dans ce cas. Ainsi, un acide inorganique volatil tel que HCl (ou un acide organique tel que l'acide acétique) risquerait de s'évaporer de la matrice sol-gel, ce qui ferait perdre en efficacité, voire empêcherait le capteur de fonctionner. Cet inconvénient n'est pas présent pour les acides dérivés d'un polymère, comme l'acide polystyrène sulfonique, car ceux-ci ne sont pas volatils.

Enfin, un autre avantage majeur des acides dérivés de polymères concerne le déploiement des chaînes de polymère acide dans les pores de petite taille de la matrice sol-gel. Ce déploiement de groupes fonctionnels acides dans les pores permet d'une part d'acidifier chaque pore et d'autre part de restreindre fortement la diffusion du polymère dans le réseau poreux, empêchant celui-ci de migrer vers la surface du capteur.

- Les avantages de cette méthode comparativement à celle actuellement utilisée avec le piégeage de l'hydrazine dans des cartouches suivi d'une dérivation en différé avec le réactif benzaldéhyde sont nombreux. Le capteur selon l'invention permet : la mesure directe de l'hydrazine sur site ;

- la mesure de l'hydrazine notamment via la mesure de l'absorbance du composé d'addition DMACA-$N_2H_4$ à 388 nm (Figure 1) ;

- la mesure de l'hydrazine par prélèvement de l'air à analyser avec un débit pouvant varier de 10 à 600 mL·min$^{-1}$, par exemple un débit de 200 mL·min$^{-1}$, la durée de prélèvement pouvant varier en fonction de la concentration du gaz à détecter, par exemple entre 60 min et 5 min, ici respectivement pour 1 et 100 ppb d'hydrazine détectés ;

- la mesure sélective de l'hydrazine dans la gamme de concentration 1-85 ppb en présence d'interférents tels que $NH_3$, l'éthanolamine ($NH_2EtOH$) ou la morpholine, lorsque la concentration totale des interférents potentiels est inférieure à 200 fois la concentration d'hydrazine ([$N_2H_4$]) ;

- la mesure sélective de l'hydrazine dans des mélanges gazeux dont le taux d'humidité relative varie entre 25 et 100 %.

De façon avantageuse, le capteur nanoporeux selon l'invention est caractérisé par une grande surface spécifique d'adsorption. En effet, il présente une surface spécifique d'adsorption de 700 à 2500 m$^2$.g$^{-1}$, de préférence de 800 à 2000 m$^2$.g$^{-1}$.

La surface spécifique d'adsorption, le volume poreux et la distribution des tailles de pores sont déterminées par analyse de l'isotherme d'adsorption-désorption à l'azote liquide avec le modèle DFT (Théorie de la Fonctionnelle de Densité). La méthode BET (Brunauer, Emmett et Teller) est une méthode analytique qui permet de déduire la surface spécifique d'adsorption.

Le capteur nanoporeux selon l'invention présente préférentiellement un volume poreux de 0,1 à 0,9 cm$^3$.g$^{-1}$, de préférence de 0,2 à 0,8 cm$^3$.g$^{-1}$, encore plus préférentiellement de 0,2 à 0,6 cm$^3$.g$^{-1}$. Le volume poreux représente le volume occupé par les pores par gramme de capteur. Le volume poreux du matériau est obtenu à partir de l'isotherme d'adsorption de l'azote à la température de l'azote liquide.

De façon avantageuse, le capteur nanoporeux selon l'invention présente une proportion de micropores supérieure à 75%, de préférence supérieure à 80%, de préférence allant de 85% à 95%, le complément à 100% correspondant à la proportion de mésopores.

Selon la définition IUPAC *(IUPAC. Compendium of Chemical Terminology, 2nd ed. (the "Gold Book"). Compiled by A. D. McNaught and A. Wilkinson. Blackwell Scientific Publications, Oxford (1997). Version en ligne (2019-) créée par S. J. Chalk. ISBN 0-9678550-9-8. https://doi.org/10.1351/goldbook)*, on entend par micropores les pores avec une largeur n'excédant pas 2 nm, par mésopores les pores présentant une largeur entre 2 et 50 nm. Les pores ayant une largeur supérieure à 50 nm sont des macropores selon la même référence IUPAC.

Le capteur nanoporeux selon l'invention, présente de façon préférentielle une proportion de mésopores inférieure à 25%, de préférence inférieure à 20%, plus préférentiellement allant de 5% à 15%, le complément à 100% correspondant à la proportion de micropores.

En particulier, le capteur nanoporeux selon l'invention peut présenter des micropores ayant un diamètre allant de 0,3 à 2 nm, de préférence de 0,5 à 2 nm.

Le capteur nanoporeux selon l'invention, peut également être caractérisé en ce qu'il présente de façon avantageuse des mésopores ayant un diamètre compris entre 2 et 20 nm, de préférence entre 2 et 15 nm.

Le capteur nanoporeux selon l'invention, présente de façon préférée un ratio r des concentrations d'acide polystyrène sulfonique et de 4-(diméthylamino)-cinnamaldéhyde de 1 à 20, de préférence de 2 à 15, encore plus préférentiellement 10.

Ce ratio est calculé à partir des concentrations molaires de l'acide polystyrène sulfonique et du 4-(diméthylamino)-cinnamaldéhyde (r = [H$^+$] / [DMACA]). Pour ce calcul, on utilise la masse molaire du monomère d'acide polystyrène sulfonique (M = 184 g/mol) afin de convertir la concentration massique de l'acide polystyrène sulfonique (PSS) en concentration molaire. Par exemple, une concentration de PSS de 9,2 g.L$^{-1}$ correspond à une concentration de 0,05 mol. L$^{-1}$ (= 9,2 / 184). Ainsi avec une concentration en DMACA de 5.10$^{-3}$ mol. L$^{-1}$ et une concentration en PSS de 9,2 g.L$^{-1}$ (0,05 mol. L$^{-1}$), le ratio r est égal à 10.

Le ratio des concentrations d'acide polystyrène sulfonique et de 4-(diméthylamino)-cinnamaldéhyde peut également être exprimé avec un ratio r' des concentrations massiques des deux espèces (r' = [H$^+$] / [DMACA]), ce ratio r' allant de 1 à 20, de préférence de 2 à 15, encore plus préférentiellement 11. Par exemple, avec une concentration en DMACA de 5.10$^{-3}$ mol.L$^{-1}$ correspondant à 0,876 g.L$^{-1}$ (avec M = 175,23 g.mol$^{-1}$) et une concentration en PSS de 9,2 g.L$^{-1}$, le ratio r' est égal à 10,5.

La présente invention a également pour objet le procédé de préparation du capteur nanoporeux selon l'invention, ledit procédé comprenant les étapes suivantes:

a. Synthèse d'un sol à partir d'un précurseur organosilylé choisi, la synthèse étant effectuée dans un solvant, ledit solvant comprenant de l'eau, en présence de 4-(diméthylamino)-cinnamaldéhyde et d'acide polystyrène sulfonique ;

b. Moulage du sol obtenu à l'étape a), suivi de sa gélification de façon à obtenir un gel ;

c. Séchage du gel obtenu à l'obtenu à l'étape b), suivi de son démoulage de façon à obtenir un capteur nanoporeux.

Selon un mode de réalisation préféré du procédé selon l'invention, le précurseur organosilylé est choisi parmi les précurseurs à hydrolyse et condensation rapides et ne comportant pas de longues chaînes hydrophobes tels que le tétraméthoxysilane Si(OCH$_3$)$_4$), le méthyltriméthoxysilane CH$_3$Si(OCH$_3$)$_3$, l'éthyltriméthoxysilane (C$_2$H$_5$)Si(OCH$_3$)$_3$, le 3-aminopropyltriéthoxysilane (C$_3$H$_6$NH$_2$)Si (OC$_2$H$_5$)$_3$, le 3-aminopropyltriméthoxysilane (C$_3$H$_6$NH$_2$)Si(OCH$_3$)$_3$), le (3-(méthylamino)propyl)triméthoxysilane (C$_3$H$_6$NHCH$_3$)Si(OCH$_3$)$_3$, le 3-carboxypropyltriéthoxysilane (C$_3$H$_6$CO$_2$H)Si(OC$_2$H$_5$)$_3$, le 3-carboxypropyltriméthoxysilane (Si(C$_3$H$_6$CO$_2$H)(OCH$_3$)$_3$), le tétraéthoxysilane et leur mélange. Le précurseur est préférentiellement choisi parmi le le tétraméthoxysilane (TMOS), le tétraéthoxysilane (TEOS) et leur mélange.

L'étape a) du procédé selon l'invention est préférentiellement réalisée dans un solvant pouvant être de l'eau ou un mélange eau/alcool, l'alcool étant de préférence un alcool aliphatique en C1 à C6, plus préférentiellement du méthanol ou de l'éthanol, ou un mélange d'eau avec un solvant choisi parmi l'acétone, le formamide, la méthyléthylcétone.

Dans le procédé selon l'invention, l'étape a) de synthèse du sol est avantageusement effectuée dans un premier temps par mélange d'un précurseur organosilylé choisi parmi le tétraméthoxysilane Si(OCH$_3$)$_4$), le méthyltriméthoxysilane CH$_3$Si(OCH$_3$)$_3$, l'éthyltriméthoxysilane (C$_2$H$_5$)Si(OCH$_3$)$_3$, le 3-aminopropyltriéthoxysilane (C$_3$H$_6$NH$_2$)Si (OC$_2$H$_5$)$_3$, le 3-aminopropyltriméthoxysilane (C$_3$H$_6$NH$_2$)Si(OCH$_3$)$_3$), le (3-(méthylamino)propyl)triméthoxysilane (C$_3$H$_6$NHCH$_3$)

Si(OCH$_3$)$_3$, le 3-carboxypropyltriéthoxysilane (C$_3$H$_6$CO$_2$H)Si(OC$_2$H$_5$)$_3$, le 3-carboxypropyltriméthoxysilane (Si(C$_3$H$_6$CO$_2$H)(OCH$_3$)$_3$), le tétraéthoxysilane et leur mélange, avec du 4-(diméthylamino)-cinnamaldéhyde dans le solvant, ledit solvant comprenant de l'eau, puis ajout dans un second temps d'acide polystyrène sulfonique.

L'ajout d'acide polystyrène sulfonique est de préférence réalisé goutte-à-goutte car la dissolution de l'acide dans le mélange est exothermique. Alternativement, l'ajout de la solution d'acide polystyrène sulfonique peut être réalisé par ajout sur le mélange du précurseur organosilylé et du 4-(diméthylamino)-cinnamaldéhyde dans le solvant, ce mélange étant maintenu à une température inférieure à 10°C.

Selon un mode de réalisation préféré, l'étape a) du procédé selon l'invention est caractérisée en ce que le rapport molaire du précurseur organosilylé sur le solvant va de 1/20 à 1/2, de préférence de 1/18 à 1/3, encore plus préférentiellement de 1/16 à 1/4.

De façon avantageuse, l'étape a) du procédé selon l'invention est réalisée à température ambiante, le terme température ambiante désignant une température d'environ 20-22°C.

Selon un mode de réalisation préféré, le mélange obtenu au terme de l'étape a) est maintenu sous agitation avant l'étape b) de gélification. De préférence, cette agitation est réalisée à température ambiante pendant une durée allant de 2 à 48 heures, de préférence 24 heures.

L'étape b) de gélification peut être réalisée après coulage dans un moule du mélange obtenu au terme de l'étape a). Lorsqu'un moule a ainsi été utilisé, le procédé selon l'invention peut alors comporter après l'étape c) de séchage, une étape de démoulage du capteur nanoporeux.

L'étape b) de gélification peut être avantageusement réalisée sous un taux d'humidité relative de 100%, à une température comprise entre 20 et 25°C, de préférence 22°C. Cette étape b) a, de préférence, une durée de 1 à 15 jours, de préférence de 1 à 5 jours, encore plus préférentiellement 2 jours.

L'étape c) de séchage peut être avantageusement réalisée dans une enceinte fermée, par exemple un dessiccateur, dont l'humidité et la température sont de préférence contrôlées.

L'étape c) de séchage est de façon avantageuse réalisée en balayant l'enceinte fermée avec un flux de gaz inerte et humide, de préférence de l'argon. L'humidité relative du flux de gaz appliqué peut avantageusement être contrôlée, en étant initialement de 100%, puis diminuée par palier jusqu'à atteindre environ 25%. L'humidité dans l'enceinte fermée peut ainsi diminuer au cours de l'étape de séchage pour atteindre 25 à 28 % d'humidité relative.

Cette étape c) a, de préférence, une durée de 15 à 60 jours, de préférence de 20 à 40 jours.

Après séchage, le capteur nanoporeux obtenu est préférentiellement stocké à l'abri de la lumière, à une température allant de 2 à 10°C, de préférence de 4 à 8°C, encore plus préférentiellement 6°C.

Le capteur nanoporeux selon l'invention et pouvant être obtenu par le procédé selon l'invention a avantageusement une forme parallélépipédique. Celui-ci présente avantageusement des dimensions de l'ordre du millimètre, par exemple une hauteur de 7,5 à 10,3 mm, une largeur de 4,8 à 6,4 mm et une épaisseur de 1 à 1,3 mm.

La présente invention a également pour objet un procédé de détection dans un échantillon gazeux à analyser d'au moins un composé de type amine, ledit composé étant choisi parmi l'hydrazine, l'éthanolamine, l'ammoniac et la morpholine, à l'aide d'un capteur nanoporeux selon l'invention, ledit procédé comprenant les étapes de :

    a. mise en contact dudit échantillon gazeux à analyser avec ledit capteur nanoporeux,

    b. détection sur ledit capteur nanoporeux du ou des dit(s) composé(s) de type amine dans l'échantillon gazeux à analyser.

Ce procédé permet avantageusement de mesurer sélectivement un composé de type amine, en particulier l'hydrazine, de manière directe, dans une gamme de concentration de 1 à 100 ppb dans de l'air sec à très humide et en présence d'interférents dont la concentration totale peut être au maximum 200 fois plus élevée que celle de l'amine à détecter, en particulier l'hydrazine.

Au sens de la présente invention, on entend par air sec, un air dont le taux d'humidité est inférieur ou égal à 25%.

Ainsi, le procédé selon l'invention permet d'effectuer des mesures sur un échantillon gazeux dont le taux d'humidité relative varie entre 25 et 100 %, de préférence de 30 à 100 %.

Le procédé selon l'invention présente donc de réels avantages par rapport aux procédés de l'art antérieur qui ne permettent pas d'effectuer cette mesure de manière directe, quel que soit le taux d'humidité, sur cette gamme de concentration et en présence d'interférents.

Outre la détection, le procédé selon l'invention peut également permettre la détermination dans un échantillon gazeux à analyser de la concentration d'au moins un composé de type amine, ledit composé étant choisi parmi l'hydrazine, l'éthanolamine, l'ammoniac et la morpholine. Ainsi, selon un mode de réalisation particulier, la présente invention porte sur un procédé de détection et de quantification dans un échantillon gazeux à analyser d'au moins un composé de type amine, ledit composé étant choisi parmi l'hydrazine, l'éthanolamine, l'ammoniac et la morpholine, à l'aide d'un capteur nanoporeux selon l'invention, ledit procédé comprenant les étapes de :

a. mise en contact dudit échantillon gazeux à analyser avec ledit capteur nanoporeux,

b. détection et quantification sur ledit capteur nanoporeux du ou des dit(s) composé(s) de type amine dans l'échantillon gazeux à analyser.

L'étape b) du procédé selon l'invention peut notamment comprendre une étape de mesure de l'absorbance du capteur nanoporeux en fonction du temps.

La méthode analytique qui permet la détection et la détermination des teneurs de l'hydrazine et des interférents porteurs d'une fonction amine primaire ou secondaire par des mesures d'absorbance en fonction du temps implique la connaissance des spectres d'absorption du mélange 4-(diméthylamino)-cinnamaldéhyde (DMACA)/ acide polystyrène sulfonique (PSS) en présence des composés de type amine visés pour différentes concentrations des dits composés aminés.

A partir de ces spectres d'absorption, des courbes d'étalonnage représentant l'évolution temporelle de l'absorbance en fonction de la concentration de chacun des dits composés de type amines peuvent être établies.

Ainsi, lorsque le capteur selon l'invention est mis en présence d'un échantillon gazeux à analyser, le résultat de mesure d'absorbance obtenu en fonction du temps peut être corrélé avec les courbes d'étalonnages précédemment établies, ce qui conduit à la détermination de la présence ou non d'un composé de type amine et optionnellement à la détermination de sa concentration.

La détection et optionnellement la quantification de l'hydrazine, l'éthanolamine, l'ammoniac ou la morpholine par des mesures d'absorbance est permise grâce à la réaction ayant lieu entre ces composés et le 4-(diméthylamino)-cinnamaldéhyde (DMACA) en présence d'acide polystyrène sulfonique (PSS)) qui joue le rôle de catalyseur et qui aboutit à la formation de complexes qui présentent des spectres d'absorption détectables dans le domaine de l'UV-visible, par exemple à l'aide d'un spectrophotomètre.

Ainsi, la réaction entre $N_2H_4$ et DMACA catalysée en présence de l'acide polystyrène sulfonique donne lieu à la formation des complexes d'addition, 1DMACA-$N_2H_4$ et 2DMACA-$N_2H_4$ selon les équations représentées dans la Figure 1.

La formation de ces deux complexes 1DMACA-$N_2H_4$ et 2DMACA-$N_2H_4$ peut être visualisée par les pics d'absorbance respectivement à 388 et 558 nm ([Fig.2]).

Dans le cadre de la présente invention, c'est le pic à 388 nm qui est utilisé pour détecter et quantifier l'hydrazine car parmi les deux pics formés c'est celui qui offre la meilleure sensibilité. En effet, le complexe 2DMACA-$N_2H_4$ est formé plus lentement et en plus petite quantité que le complexe DMACA-$N_2H_4$ pour des raisons stériques et cinétiques.

En ce qui concerne l'ammoniac, DMACA($H^+$) se déprotone en présence d'une base forte tel que $NH_3$, pour former DMACA neutre absorbant intensément à 420 nm ([Fig.3]). La détection et la mesure de la concentration de $NH_3$ peuvent donc être réalisées via la mesure de la vitesse de déprotonation (correspondant au rapport de la variation d'absorbance sur le temps) en fonction de la concentration de $NH_3$ dans le mélange gazeux ([Fig.7]).

La présente invention a également pour objet l'utilisation d'un capteur nanoporeux selon l'invention pour la détection, ou la quantification ou la détection et la quantification d'au moins un composé de type amine, ledit composé étant choisi parmi l'hydrazine, l'éthanolamine, l'ammoniac et la morpholine.

Le capteur nanoporeux selon l'invention peut être utilisé en mode dynamique ou en mode statique. Ainsi le capteur nanoporeux peut être placé dans un flux gazeux à analyser et mis en circulation par un circuit fluidique ou alternativement le capteur peut être placé dans une enceinte comprenant l'échantillon gazeux à analyser.

La présente invention a également pour objet un dispositif (1) de détection d'au moins un composé de type amine, ledit composé étant choisi parmi l'hydrazine, l'éthanolamine, l'ammoniac et la morpholine, dans un échantillon gazeux à analyser, ledit dispositif comprenant une cellule (11) renfermant un capteur nanoporeux selon l'invention, ladite cellule comprenant :

- une entrée de gaz (111) ;

- une sortie de gaz (112);

- une entrée optique (113);

- une sortie optique (114).

De façon avantageuse, le dispositif selon l'invention comporte un circuit fluidique adapté pour faire circuler l'échantillon gazeux à analyser à travers la cellule (11) en générant un flux gazeux de l'entrée de gaz (111) vers le capteur nanoporeux puis vers la sortie de gaz (112).

Selon un mode de réalisation préféré, le circuit fluidique selon l'invention comprend un régulateur de débit (2) adapté pour réguler le débit du flux gazeux. Le régulateur de débit (2) peut être composé d'une vanne d'arrêt (21) et d'une vanne pointeau de régulation (22).

De façon avantageuse, le dispositif selon l'invention comprend en outre une source de lumière (3) et un spectrophotomètre (4), dans lequel l'entrée optique (113) de la cellule est reliée à la source de lumière (3) et la sortie optique (114) de la cellule est reliée au spectrophotomètre (4). La source de lumière (3) et le spectrophotomètre (4) peuvent ainsi constituer la partie d'analyse optique du dispositif.

Selon un mode de réalisation préféré, le circuit fluidique du dispositif selon l'invention comprend en outre une sonde de température adaptée pour mesurer la température du flux gazeux.

De façon avantageuse, le dispositif selon l'invention comprend en outre une sonde d'humidité adaptée pour mesurer l'humidité du flux gazeux.

De façon avantageuse, le dispositif selon l'invention comprend en outre un débitmètre (5) adapté pour mesurer le débit du flux gazeux.

Le circuit fluidique peut ainsi tenir compte :

- du flux de mélange gazeux nécessaire à l'exposition des capteurs, ce flux pouvant être varié par exemple entre 50 et 600 mL/min selon le capteur,

- de la mesure de la température et de l'humidité relative du mélange gazeux,

- d'un mode de purge du circuit de mesure.

Le dispositif selon l'invention peut ainsi fonctionner de la manière suivante : l'air ou l'échantillon gazeux à analyser peut être aspiré à l'aide d'une pompe (6), celle-ci pouvant être miniature, et conduit dans un premier temps à travers un répartiteur d'entrée (7) où seront mesurées la température et l'humidité de l'échantillon gazeux. Le répartiteur (7) peut ensuite distribuer l'échantillon gazeux vers 2 sorties. La pompe peut notamment avoir un débit allant de 50 à 1100ml/min.

La première sortie peut diriger l'air vers la cellule (11) renfermant le capteur nanoporeux. La cellule peut être placée en amont d'un débitmètre (5) connecté à un répartiteur de sortie (8) lui-même connecté à la pompe (6). Le débitmètre permet de fixer la vitesse du flux gazeux dans la cellule d'exposition.

La deuxième sortie du répartiteur d'entrée (7) peut être connectée au répartiteur de sortie (8) via une vanne d'arrêt (21) et une vanne pointeau de régulation (22). Cet ensemble constitue alors un circuit de fuite. Cette configuration permet de varier le débit d'exposition des capteurs (en cas d'utilisation de différents types de capteurs) car la pompe fonctionne en continu à un débit fixe, par exemple de 1.1 L/min. La vanne d'arrêt permet de couper le circuit de fuite pour purger le circuit de mesure en cas de besoin.

La partie d'analyse optique comporte une source de lumière (3) qui peut venir sonder le capteur et un spectrophotomètre (4), ce dernier pouvant être miniature, qui va recueillir la lumière transmise par le capteur et la transformer en courant électrique. Le spectrophotomètre (4) peut fonctionner à des longueurs d'onde allant de l'UV à l'IR en passant par le visible, la longueur d'onde étant choisie en fonction de la mesure à effectuer.

La source de lumière (3) peut être constituée de 2 LEDs reliées par des fibres optiques ou d'une lampe miniature. La source de lumière peut être une source UV ou visible ou UV-visible.

Pour la détection de l'hydrazine à 388 nm, de $NH_3$ à 420 nm, d'éthanolamine à 474 nm ou de morpholine à 490 nm, la combinaison d'une LED UV et d'une LED visible permet d'obtenir la gamme de longueur d'onde 380-800 nm. D'autres choix de LEDs sont envisageables selon la réponse optique du capteur. La lumière est véhiculée vers la cellule d'exposition par exemple à l'aide d'une fibre optique. A la sortie de la cellule d'exposition, la lumière transmise est véhiculée vers le spectrophotomètre (4) miniature dont le domaine de réponse est large, par exemple de 337.5 à 822 nm.

Un exemple de dispositif selon l'invention est représenté en [Fig.4].

Selon un mode de réalisation préféré, le dispositif selon l'invention est caractérisé en ce que la cellule comprend un support de capteur nanoporeux et un boitier de forme parallélépipédique, de préférence cubique, comprenant 4 faces latérales et une face frontale formant en son centre un logement pour recevoir le support de capteur nanoporeux,

deux faces latérales opposées présentant l'entrée et la sortie de gaz,

deux faces latérales opposées présentant l'entrée et la sortie optique,

la face frontale présentant une ouverture pour l'insertion du support de capteur nanoporeux dans son logement (Figure 6).

La cellule du dispositif selon l'invention peut ainsi comporter deux éléments.

Le premier élément est son corps qui peut consister en un bloc carré (Figure 6) de laiton renforcé par exemple par une couche interne d'inox, et qui peut notamment être creusé en son centre et percé de part et d'autre sur ses quatre faces. Deux tunnels perpendiculaires peuvent ainsi être créés pour servir de passages au flux gazeux et à la lumière de sonde. Ce bloc peut ainsi être muni d'une entrée et d'une sortie optiques ainsi que d'une entrée et une sortie de gaz.

Le second élément peut être une pièce amovible par exemple en inox (Figure 6) et qui peut également être percée sur les quatre faces pour le passage du flux gazeux et de la lumière. L'étanchéité des 2 tunnels entre le bloc externe et la pièce amovible peut être réalisée à l'aide de joints toriques. La pièce amovible peut contenir en son centre le capteur nanoporeux, qui peut être par exemple un bloc monolithique transparent de dimensions maximales de l'ordre de la dizaine de millimètre, par exemple 11 x 6.5 x 2 mm. L'entrée et la sortie optiques peuvent chacune comporter une lentille pour collimater le faisceau de lumière d'analyse. L'entrée optique peut être reliée par une fibre optique à une source lumineuse UV ou Visible ou une combinaison des deux UV-visible, et la sortie optique peut être reliée au spectrophotomètre UV ou visible ou UV-visible, nécessaire à l'analyse et compatible avec la lampe, via une seconde fibre optique.

Un exemple de cellule du dispositif selon l'invention est représenté en [Fig.6].

Le capteur peut être exposé au flux gazeux sur toute sa longueur et sur les 2 faces. Le faisceau lumineux de sonde, provenant de la source de lumière et véhiculé par une fibre optique, arrive perpendiculairement au flux gazeux et est focalisé en sortie de fibre sur le capteur. La surface sondée peut être un cercle de 5,7 mm$^2$ (2,7mm de diamètre). Le parcours optique d'analyse, pouvant varier de 1 à 2 mm, correspond à l'épaisseur du capteur nanoporeux inséré.

Selon la géométrie du capteur, par exemple un disque ou un parallélépipède, la pièce amovible peut être conçue pour un bon positionnement du capteur et son exposition au flux gazeux sur sa plus grande surface.

Selon un mode de réalisation préféré, le capteur est placé entre deux demi-cylindres, creusés pour favoriser le passage du flux de gaz au-dessus et en-dessous du capteur ([Fig.5] et [Fig.6]). Le capteur est positionné à l'intersection d'un trou vertical qui laisse passer la lumière de sonde.

Selon un mode de réalisation préféré, le dispositif selon l'invention est caractérisé en ce que le support de capteur nanoporeux comprend deux orifices de passage diamétralement opposés et reliés optiquement à l'entrée et à la sortie optique.

De façon avantageuse, le dispositif selon l'invention comprend en outre un ordinateur et une batterie pour fournir une alimentation électrique audit dispositif et ainsi rendre le dispositif énergétiquement autonome.

Le dispositif selon l'invention peut également comprendre un écran permettant à l'utilisateur d'interagir avec les éléments du système par voie informatique.

Le dispositif selon l'invention peut également comprendre une carte d'alimentation et de contrôle de l'énergie.

Les éléments du dispositif composés du circuit fluidique et de la partie d'analyse optique peuvent ainsi être positionnés dans un compartiment d'un élément portable. Cet élément portable pourrait alors comporter un second compartiment renfermant un ordinateur adapté au contrôle des mesures et un troisième compartiment comportant une alimentation électrique pour faire faire fonctionner le dispositif dans son ensemble.

Ainsi, selon un mode de réalisation particulier, le dispositif selon l'invention peut être intégré dans une mallette comprenant plusieurs étages, notamment 3 étages. L'ordinateur peut par exemple se situer à l'étage supérieur de la mallette, le dispositif selon l'invention à l'étage intermédiaire et l'alimentation à l'étage inférieur.

Selon un mode de réalisation préféré, le dispositif selon l'invention est caractérisé en ce qu'il est portable.

De façon avantageuse, le dispositif selon l'invention permet la quantification du ou des dit(s) composé(s) de type amine dans l'échantillon gazeux à analyser.

**Exemples**

**Exemple 1 : Synthèse de capteurs nanoporeux selon l'invention.**

**Réactifs utilisés :**

Tetraméthylorthosilicate **(TMOS),** pureté 99%, CAS : 681-84-5, Masse molaire = 152,22 g·mol$^{-1}$ et densité d = 1,023 g.cm$^{-3}$

4-(Dimethylamino)cinnamaldehyde **(DMACA),** pureté ≥98%, CAS : 6203-18-5, Masse molaire = 175,23 g·mol$^{-1}$

Acide Polystyrène sulfonique **(PSS),** solution aqueuse à 30%, CAS : 28210-41-5, Masse molaire = 75 000 g·mol$^{-1}$ et densité d = 1,1 g.cm$^{-3}$

Acide paratoluènesulfonique **(C$_7$H$_7$-SO$_3$H),** pureté ≥98%, CAS : 6192-52-5, Masse molaire = 190,22 g·mol$^{-1}$

Eau déionisée ultra-pure.

### Exemple 1.1 : Capteur Hy1

Dans une bouteille de 1 L sont mélangés 14,2 mg de DMACA, 40,969 mL de $H_2O$ et 84,598 mL de TMOS. La solution est maintenue sous agitation à température ambiante et on ajoute goutte à goutte 0,449 mL de PSS à 30%, car la dissolution de PSS dans le mélange est exothermique. Le rapport molaire du précurseur silylé et de l'eau dans le mélange est TMOS/$H_2O$ = 1/4. Les concentrations finales respectives de DMACA et de PSS sont de 6,25 x $10^{-4}$ M et de 1,18 g·$L^{-1}$ soit [$H^+$] ~ 6,25 x $10^{-3}$ M.

Le sol est maintenu sous agitation pendant 24H à température ambiante puis est versé dans un moule en polypropylène contenant 350 puits de 0,3 $cm^3$ de volume. Le moule est mis dans un dessiccateur de 10L maintenu à une humidité relative de 100% jusqu'à gélification du Sol. Cette étape dure 2 jours à 22°C pour Hy1. Le séchage des gels est alors réalisé en balayant le dessiccateur avec un flux d'Ar humide à 300 mL·$min^{-1}$. L'humidité relative du flux d'Ar, initialement de 100%, est diminuée par palier à 80, 50 puis 0%. Le temps de séchage prend environ 1 mois à 22°C, pendant lesquels l'humidité dans le dessiccateur diminue jusqu'à atteindre 25 % à 28% d'humidité relative. Le moule est alors retiré du dessiccateur. Après démoulage, on obtient des capteurs de forme parallélépipédique de dimensions 9,6(H)*6,0(L) *1,26(épaisseur) mm pour une solution initiale de 0,3 mL. Le volume final des capteurs obtenus a rétréci d'un facteur 4,2. Les capteurs sont conservés au frais à 6 °C, à l'abri de la lumière.

### Exemple 1.2 : Capteur Hy2

Même mode opératoire que Hy1 avec 14,2 mg de DMACA, 84,598 mL de TMOS, 40,969 mL de $H_2O$ déionisée et 0,673 mL de PSS 30%. Le rapport molaire du précurseur silylé et de l'eau dans le mélange est TMOS/$H_2O$ = 1/4. Les concentrations finales respectives de DMACA et de PSS sont de 6,25 x $10^{-4}$ M et de 1,77 g·$L^{-1}$ soit [$H^+$] ~ 9,38 x $10^{-3}$ M. La durée de gélification des capteurs dans le dessiccateur maintenu à HR=100% est de 2 jours à 22°C. La durée du séchage par paliers de HR= 80, 50 à 0% prend environ 40 jours à 22°C, pendant lesquels l'humidité dans le dessiccateur diminue jusqu'à atteindre 25 % à 28% d'humidité relative. Après démoulage, on obtient des capteurs de dimensions 9,7(H)*6,1(L) *1,26(épaisseur) mm avec un facteur de rétrécissement de 4. Les capteurs sont conservés au frais à 6 °C, à l'abri de la lumière.

### Exemple 1.3 : Capteur Hy3

Même mode opératoire que Hy1 avec 28.5 mg de DMACA, 84,598 mL de TMOS, 40,969 mL de $H_2O$ déionisée et 0,898 mL de PSS 30%. Le rapport molaire du précurseur silylé et de l'eau dans le mélange est TMOS/$H_2O$ = 1/4. Les concentrations finales respectives de DMACA et de PSS sont de 1,25 x $10^{-3}$ M et de 2,34 g·$L^{-1}$ soit [$H^+$] ~ 1,25 x $10^{-2}$ M. La durée de gélification des capteurs dans le dessiccateur maintenu à HR=100% est de 2 jours à 22°C. La durée du séchage par paliers de HR= 80, 50 à 0% prend environ 1 mois à 22°C, pendant lesquels l'humidité dans le dessiccateur diminue jusqu'à atteindre 25 % à 28% d'humidité relative. Après démoulage, on obtient des capteurs de dimensions 9,5(H)*6,1(L) *1,24(épaisseur) mm avec un facteur de rétrécissement de 4,2. Les capteurs sont conservés au frais à 6 °C, à l'abri de la lumière.

### Exemple 1.4 : Capteur Hy4

Même mode opératoire que Hy1 avec 113 mg de DMACA, 81,496 mL de $H_2O$ et 43,91 mL de TMOS et 3,563 mL de PSS à 30%. Le rapport molaire du mélange du précurseur silylé et de l'eau est TMOS/$H_2O$ = 1/16. Les concentrations finales respectives de DMACA et de PSS sont de 5 x $10^{-3}$ M et de 9,2 g·$L^{-1}$ soit [$H^+$] ~ 5 x $10^{-2}$ M. La gélification des capteurs dans le dessiccateur maintenu à HR=100% a lieu au bout de 5 jours à 22°C. La durée du séchage par paliers de HR= 80, 50 à 0% prend environ 1,5 mois à 22°C, pendant lesquels l'humidité dans le dessiccateur diminue jusqu'à atteindre 25 % à 28% d'humidité relative. Après démoulage, on obtient des capteurs de dimensions 8,37(H)*4,94(L)*1,03(épaisseur) mm avec un facteur de rétrécissement de 7. Les capteurs sont conservés au frais à 6 °C, à l'abri de la lumière.

### Exemple 1.5 : Capteur Hy5

Même mode opératoire que Hy1 avec 87,9 mg de DMACA, 33,777 mL de TMOS, 13,587 mL de $H_2O$ et 2,77 mL de PSS 30%. Le rapport molaire du précurseur silylé et de l'eau dans le mélange est TMOS/$H_2O$ = 1/4. Les concentrations finales respectives de DMACA et de PSS sont de $1 \times 10^{-2}$ M et de 18,4 g·$L^{-1}$ soit [$H^+$] ~ $1 \times 10^{-1}$ M. La durée de gélification des capteurs dans le dessiccateur maintenu à HR=100% est de 5 jours à 22°C. La durée du séchage par paliers de HR= 80, 50 à 0% prend environ 1 mois à 22°C, pendant lesquels l'humidité dans le dessiccateur diminue jusqu'à atteindre 25 % à 28% d'humidité relative. Après démoulage, on obtient des capteurs de dimensions 10,2(H)*6,4(L)*1,3(épaisseur) mm avec un facteur de rétrécissement de 3,5. Les capteurs sont conservés au frais à 6 °C, à l'abri de la lumière.

**Exemple 1.6 : Capteur Hy6**

Même mode opératoire que Hy1, avec 226 mg de DMACA, 43,91 mL de TMOS, 77,933 mL d'eau et 7,126 mL de PSS. Le rapport molaire du précurseur silylé et de l'eau dans le mélange est TMOS/$H_2O$ = 1/16. Les concentrations finales de DMACA et de PSS sont de $1 \times 10^{-2}$ M et de 18,4 g·$L^{-1}$ soit $[H^+] \sim 1 \times 10^{-1}$ M. La durée de gélification des capteurs dans le dessiccateur maintenu à HR=100% est de 3 jours à 22°C. La durée du séchage par paliers de HR= 80, 50 à 0% prend environ 49 jours à 22°C, pendant lesquels l'humidité dans le dessiccateur diminue jusqu'à atteindre 25 % à 28% d'humidité relative. Après démoulage, on obtient des capteurs de dimensions 8,4(H)*5,0(L)*1,05(épaisseur) mm avec un facteur de rétrécissement de 6,7.

**Exemple 1.7 : Capteur Hy7**

Dans une bouteille de 1 L sont mélangés 56,5 mg de DMACA, 21,258 mL de $H_2O$ et 10,974 mL de TMOS. La solution est maintenue sous agitation à température ambiante en ajoutant lentement 613 mg de l'acide paratoluène-sulfonique, $C_7H_7$-$SO_3H$. Lorsque l'acide est ajouté à ce mélange, celui-ci dégage la chaleur. Le rapport molaire du précurseur silylé et de l'eau est TMOS/$H_2O$ = 1/16. Les concentrations finales respectives de DMACA et de l'acide paratoluènesulfonique sont de $1 \times 10^{-2}$ M et de $1 \times 10^{-1}$ M.
Le sol est maintenu sous agitation pendant 3H à température ambiante puis est versé dans un moule en polypropylène. Le moule est mis dans un dessiccateur de 10L maintenu à une humidité relative de 100% jusqu'à gélification du Sol. Cette étape dure 5 jours à 22°C. Le séchage est réalisé avec un balayage du dessiccateur avec un flux d'Ar de 300 mL·$min^{-1}$, en diminuant progressivement l'humidité relative dans le dessiccateur de 100% à 80%, 50% puis 0% HR. Le temps de séchage prend environ 1 mois à 22°C, pendant lesquels l'humidité dans le dessiccateur diminue jusqu'à atteindre 25 % à 28% d'humidité relative. Le moule est retiré du dessiccateur. Après démoulage, on obtient des capteurs de forme parallélépipédique de dimensions 7,57(H)*4,8(L)*1,0(épaisseur) mm avec un facteur de rétrécissement de 8,2. Les capteurs sont conservés au frais à 6 °C, à l'abri de la lumière.

**Exemple 1.8 : capteur Hy8**

Même mode opératoire que Hy1 avec 1,139 g de DMACA, 84,598 mL de TMOS, 5,054 et 35,916 mL PSS 30%. Les concentrations finales respectives de DMACA et de PSS sont de $5 \times 10^{-2}$ M et de 95,2 g·$L^{-1}$ soit $[H^+] \sim 5 \times 10^{-1}$ M. La durée de gélification des capteurs dans le dessiccateur maintenu à HR=100% est de 4 jours à 22°C. La durée du séchage par paliers de HR= 80, 50 à 0% prend environ 1 mois à 22°C, pendant lesquels l'humidité dans le dessiccateur diminue jusqu'à atteindre 25 % à 28% d'humidité relative. Après démoulage, on obtient des capteurs de dimensions 11,2(H)*6,4(L)*1,2(épaisseur) mm avec le facteur de rétrécissement de 3,4.

**Exemple 1.9 : capteur Hy9**

Même mode opératoire que Hy1 avec 596,5 mg de DMACA, 84,598 mL de TMOS, 23,012 mL d'eau et 17,958 mL de PSS 30%. Le rapport molaire du précurseur silylé et de l'eau est TMOS/$H_2O$ = 1/4. Les concentrations finales respectives de DMACA et de PSS sont de $2,5 \times 10^{-2}$ M et de 47,6 g·$L^{-1}$ soit $[H^+] \sim 2,5 \times 10^{-1}$ M. La durée de gélification des capteurs dans le dessiccateur maintenu à HR=100% est de 2 jours à 22°C. La durée du séchage par paliers de HR= 80, 50 à 0% prend environ 1 mois à 22°C, pendant lesquels l'humidité dans le dessiccateur diminue jusqu'à atteindre 25 % à 28% d'humidité relative. Après démoulage, on obtient des capteurs de dimensions 10,24(H)*6,5(L)*1,25(épaisseur) mm avec un facteur de rétrécissement de 3,6.
Les propriétés de porosité des capteurs nanoporeux telles que la surface spécifique d'adsorption, le volume poreux ou encore les distributions de tailles des micropores et des mésopores ont été déterminées à partir de l'établissement d'isothermes d'adsorption-désorption de $N_2$ à la température de $N_2$ liquide. Le tableau ci-dessous regroupe ces données.

[Tableau 1]

| Capteur | Formulation (TMOS/$H_2O$) | Concentration de réactifs dans le sol (mol.$L^{-1}$) | | | $\dfrac{V_{sol}}{V_{solide}}$ | Propriétés de porosité | | | |
| --- | --- | --- | --- | --- | --- | --- | --- | --- | --- |
| | | [DMACA] | [monomère PSS] = [$H^+$] | $\dfrac{[H^+]}{[DMACA]}$ | | $S_{DFT}$/ $m^2.g^{-1}$ | $V_{pore}$ / $cm^3.g^{-1}$ | % de $\mu$pore Taille en Å | % de mésopore Taille en Å |
| Hy1 | 1/4 en mole | $6{,}25 \times 10^{-4}$ | $6{,}25 \times 10^{-3}$ | 10 | 4,2 | 1794 | 0,33 | 100% 5< d <19 | 0% |
| Hy2 | 1/4 en mole | $6{,}25 \times 10^{-4}$ | $9{,}38 \times 10^{-3}$ | 15 | 4,0 | 1929 | 0,44 | 95% 5< d <20 | 5% 20< d <88 |
| Hy3 | 1/4 en mole | $1{,}25 \times 10^{-3}$ | $1{,}25 \times 10^{-2}$ | 10 | 4,2 | 1878 | 0,4 | 97% 5< d <20 | 3% 20< d <65 |
| Hy4 | 1/16 en mole | $5 \times 10^{-3}$ | $5 \times 10^{-2}$ | 10 | 7,0 | 1478 | 0,51 | 82% 5< d <12 | 18% 20< d <131 |
| Hy5 | 1/4 en mole | $1 \times 10^{-2}$ | $1 \times 10^{-1}$ | 10 | 3,5 | 1593 | 0,42 | 90% 5< d <20 | 10% 20< d <102 |
| Hy6 | 1/16 en mole | $1 \times 10^{-2}$ | $1 \times 10^{-1}$ | 10 | 6,7 | 1414 | 0,42 | 86% 5< d <20 | 14% 20< d <88 |
| Hy7 | 1/16 en mole | $1 \times 10^{-2}$ | $1 \times 10^{-1}$ ($C_7H_7$-$SO_3H$) | 10 | 8,2 | 853 | 0,24 | 88% 6< d <20 | 12% 20< d <61 |
| Hy8 | 1/4 en mole | $5 \times 10^{-2}$ | $5 \times 10^{-1}$ | 10 | 3,4 | 1778 | 0,52 | 89% 8< d <13 | 11% 20< d <131 |
| Hy9 | 1/4 en mole | $2{,}5 \times 10^{-2}$ | $2{,}5 \times 10^{-1}$ | 10 | 3,6 | 1608 | 0,49 | 87% 5< d <20 | 13% 20< d <107 |

Les pourcentages de micropores et de mésopores donnés ici correspondent à la distribution de la surface d'adsorption en fonction du diamètre des pores. Ce pourcentage serait différent si on considère la distribution du volume poreux en fonction du diamètre des pores.

**Exemple 2 : Réponse du capteur Hy1 à NH$_3$**

Le capteur Hy1 a été exposé à un mélange gazeux humide (Taux d'humidité relative HR = 50%) contenant 5 ppm de NH$_3$. NH$_3$ ne réagit pas avec DMACA(H$^+$) mais induit une déprotonation de DMACA(H$^+$) pour former DMACA neutre. Dans le matériau poreux, DMACA neutre présente une bande d'absorption large dans l'UV-proche visible avec le maximum centré à 420 nm **(Erreur ! Source du renvoi introuvable.).** La courbe d'étalonnage du capteur Hy1 correspondant à la vitesse de formation de DMACA neutre en fonction de la concentration de NH$_3$ est montrée dans la Figure 7.

**Exemple 3 : Réponse de capteur Hy3 à N$_2$H$_4$. Etablissement d'une courbe d'étalonnage de N$_2$H$_4$ gazeux**

Les capteurs Hy3 sont exposés à différentes concentrations de N$_2$H$_4$ dans une large plage de concentration de 1 à 114 ppb. L'humidité relative des mélanges gazeux a été maintenue fixe à 50%. Pour chaque exposition à une teneur donnée de N$_2$H$_4$, la vitesse de formation du complexe 1DMACA-N$_2$H$_4$ à 388 nm a été déduite. En traçant la vitesse de formation du complexe 1DMACA-N$_2$H$_4$ en fonction de la concentration de N$_2$H$_4$, on obtient une courbe d'étalonnage pour la détection de N$_2$H$_4$ à 388 nm. Un exemple de courbe d'étalonnage de N$_2$H$_4$ établie pour le capteur Hy3 est donné dans la **Erreur ! Source du renvoi introuvable..**

La **Erreur ! Source du renvoi introuvable.** montre la courbe de calibration de N$_2$H$_4$ réalisée en exposant les capteurs Hy3 avec des durées de stockage variant de cinq mois à quinze mois. On obtient une variation linéaire des vitesses de formation du complexe 1DMACA-N$_2$H$_4$ en fonction de la concentration de N$_2$H$_4$. La limite de détection est de 1 ppb pour un volume gazeux sondé de 12L (60 minutes, 200 mL·min$^{-1}$ et $\Delta$Abs = 0,02). La durée d'exposition peut être diminuée en augmentant le débit de flux du mélange gazeux à analyser.

**Exemple 4 : Réponse des capteurs Hy3 à N$_2$H$_4$, à différentes humidités relatives**

L'effet de l'humidité relative des mélanges gazeux sur la courbe d'étalonnage de N$_2$H$_4$ du capteur Hy3 est étudié ([Fig.9]). A cet effet, les capteurs Hy3 sont exposés à différentes concentrations de N$_2$H$_4$ dans des mélanges gazeux à 30, 50 et 80% d'HR.

**Exemple 5 : Réponse du capteur Hy3 à N$_2$H$_4$ en présence d'un interférent potentiel, NH$_2$EtOH**

La réponse du capteur Hy3 à N$_2$H$_4$ en présence de **NH$_2$EtOH** est étudiée ([Fig.10]). A cet effet, les capteurs Hy3 sont exposés à des mélanges gazeux contenant N$_2$H$_4$ + NH$_2$EtOH.

**Exemple 6 : Réponse du capteur Hy3 à N$_2$H$_4$ en présence d'un interférent potentiel, morpholine**

La réponse du capteur Hy3 à N$_2$H$_4$ en présence de morpholine est étudiée ([Fig.11]). A cet effet, les capteurs Hy3 sont exposés à des mélanges gazeux contenant N$_2$H$_4$ + morpholine.

**Exemple 7 : Réponse du capteur Hy3 à N$_2$H$_4$ en présence d'un interférent potentiel, NH$_3$**

La réponse du capteur Hy3 à N$_2$H$_4$ en présence de NH$_3$ est étudiée ([Fig.12]). A cet effet, les capteurs Hy3 sont exposés à des mélanges gazeux contenant N$_2$H$_4$ + NH$_3$.

**Exemple 8 : Réponse du capteur Hy3 à N$_2$H$_4$ en présence des deux interférents potentiels, NH$_3$ et NH$_2$EtOH**

La réponse du capteur Hy3 à N$_2$H$_4$ en présence des deux interférents potentiels, NH$_3$ et NH$_2$EtOH, est étudiée ([Fig.13]). A cet effet, les capteurs Hy3 sont exposés à des mélanges gazeux contenant N$_2$H$_4$ + NH$_3$ + NH$_2$EtOH.

**Exemple 9 : Réponse du capteur Hy3 à N$_2$H$_4$ en présence de deux interférents potentiels, NH$_3$ et morpholine**

La réponse du capteur Hy3 à N$_2$H$_4$ en présence des interférents, NH$_3$ et morpholine, est étudiée ([Fig.14]). A cet effet, les capteurs Hy3 sont exposés à des mélanges gazeux contenant N$_2$H$_4$ + NH$_3$ + morpholine.

## EP 4 553 496 B1

**Exemple 10 : Comparaison des réponses des capteurs Hy1, Hy2 et Hy3 à 30 ppb de $N_2H_4$**

Les réponses des capteurs Hy1, Hy2 et Hy3 à $N_2H_4$ sont étudiées ([Fig.15]). A cet effet, les capteurs Hy1, Hy2 et Hy3 sont exposés à 30 ppb de $N_2H_4$.

**Exemple 11 : comparaison des capteurs Hy8 et Hy9 à 40 ppb de $N_2H_4$**

Les réponses des capteurs Hy8 et Hy9 à $N_2H_4$ sont étudiées ([Fig.16]). A cet effet, les capteurs Hy8 et Hy9 sont exposés à 40 ppb de $N_2H_4$.

**Exemple 12 : comparaison des capteurs Hy4, Hy5, Hy6 et Hy7 à 25 ppb de $N_2H_4$**

Les réponses des capteurs Hy4, Hy5, Hy6 et Hy7 à $N_2H_4$ sont étudiées ([Fig.17]). A cet effet, les capteurs Hy4, Hy5, Hy6 et Hy7 sont exposés à 25 ppb de $N_2H_4$.

**Liste des documents cités**

[1] Fiche toxicologique n° 21 INRS http://www.inrs.fr/publications/bdd/fichetox/fiche.html?refINRS=FICHETOX_21

[2] Qualité de l'air. Air des lieux de travail. Prélèvement et analyse des vapeurs organiques. Prélèvement par pompage sur tube à adsorption et désorption au solvant. Norme NF X 43-267. La Plaine Saint Denis: AFNOR ;2004.

[3] Fiche Métropole Hydrazine M-7, http://www.inrs.fr/publications/bdd/metropol/fiche.html?refINRS=METROPOL_7

[4] Hydrazine. Method 108 In: Sampling and Analytical methods. OSHA, 1997 (https://www.osha.gov/dts/sltc/methods/organic/org108/org108.html)

[5] B. J. Meneghelli, A review of hydrazine sensors: The state of the art, ASRC Aerospace Corp., Cocoa Beach, FL, United States, 2004.

[6] K. P. Brenner, S. L. Rose-Pehrss on, Performance Evaluation of a Colorimetric Hydrazine Dosimeter, 1994.

[7] https://dodtec.com/hydrazine-mmh-dose-estimator.html

[8] https://www.chemsee.com/commercial/toxic-gas/available-products/dosimeters/hyd-009-dosimeter-for-hydrazine/

[9] Rose-Pehrsson et al., brevet US005719061A

[10] http://catalog.gasdetection.com/item/search-by-gas-type-hydrazine-s-/nalyzer-4000-series-with-digital-display-hydrazine/4180-100b

[11] https://www.raefrance.fr/produit/detecteur-cov-capteur-pid-ppbrae-3000/

[12] https://www.cbrnetechindex.com/p/3525/Smiths-Detection-Inc/Sabre-4000

[13] https://www.environics.fi/product/chempro100i/

[14] https://www.masatech.eu/portable-advanced-ion-mobility-spectrometer

[15] https://www.smithsdetection.com/products/lcd-3-3/

**Revendications**

1. Capteur nanoporeux composé d'une matrice sol-gel silicatée renfermant une composition de réactifs, ladite composition de réactifs comprenant un mélange de 4-(diméthylamino)-cinnamaldéhyde et d'acide polystyrène sulfonique.

2. Capteur nanoporeux selon la revendication 1, **caractérisé en ce qu'**il présente une surface spécifique d'adsorption de 700 à 2500 $m^2.g^{-1}$, de préférence de 800 à 2000 $m^2.g^{-1}$.

3. Capteur nanoporeux selon la revendication 1 ou 2, **caractérisé en ce qu'**il présente un volume poreux de 0,1 à 0,9 $cm^3.g^{-1}$, de préférence de 0,2 à 0,8 $cm^3.g^{-1}$, encore plus préférentiellement de 0,2 à 0,6 $cm^3.g^{-1}$.

4. Capteur nanoporeux selon l'une des revendications 1 à 3, **caractérisé en ce qu'**il présente une proportion de micropores supérieure à 75%, de préférence supérieure à 80%, plus préférentiellement allant de 85% à 95%, le complément à 100% correspondant à la proportion de mésopores.

5. Procédé de préparation d'un capteur nanoporeux selon l'une des revendications 1 à 4, ledit procédé comprenant les étapes suivantes :

   a. Synthèse d'un sol à partir d'un précurseur organosilylé, la synthèse étant effectuée dans un solvant, ledit solvant comprenant de l'eau, en présence de 4-(diméthylamino)-cinnamaldéhyde et d'acide polystyrène sulfonique ;
   b. Moulage du sol obtenu à l'étape a), suivi de sa gélification de façon à obtenir un gel ;
   c. Séchage du gel obtenu à l'étape b), suivi de son démoulage de façon à obtenir un capteur nanoporeux.

6. Procédé selon la revendication 5, **caractérisé en ce que** le précurseur organosilylé est choisi parmi le tétraméthoxysilane, le méthyltriméthoxysilane, l'éthyltriméthoxysilane, le 3-aminopropyltriéthoxysilane, le 3-aminopropyltriméthoxysilane, le (3-(méthylamino)propyl)triméthoxysilane, le 3-carboxypropyltriéthoxysilane, le 3-carboxypropyltriméthoxysilane, le tétraéthoxysilane et leur mélange.

7. Procédé de détection dans un échantillon gazeux à analyser d'au moins un composé de type amine, ledit composé étant choisi parmi l'hydrazine, l'éthanolamine, l'ammoniac et la morpholine, à l'aide d'un capteur nanoporeux selon l'une des revendications 1 à 4, ledit procédé comprenant les étapes de :

   a. mise en contact dudit échantillon gazeux à analyser avec ledit capteur nanoporeux,
   b. détection sur ledit capteur nanoporeux du ou des dit(s) composé(s) de type amine dans l'échantillon gazeux à analyser.

8. Procédé selon la revendication 7, pour lequel l'étape b) comprend en outre la quantification sur ledit capteur nanoporeux du ou des dit(s) composé(s) de type amine dans l'échantillon gazeux à analyser.

9. Utilisation d'un capteur nanoporeux selon l'une des revendications 1 à 4, pour la détection, ou la quantification ou la détection et la quantification d'au moins un composé de type amine, ledit composé étant choisi parmi l'hydrazine, l'éthanolamine, l'ammoniac et la morpholine.

10. Dispositif (1) de détection dans un échantillon gazeux à analyser d'au moins un composé de type amine, ledit composé étant choisi parmi l'hydrazine, l'éthanolamine, l'ammoniac et la morpholine, ledit dispositif comprenant :

    - une cellule (11) renfermant un capteur nanoporeux selon l'une des revendications 1 à 4 et comprenant :

       - une entrée de gaz (111) ;
       - une sortie de gaz (112) ;
       - une entrée optique (113) ;
       - une sortie optique (114).

**Patentansprüche**

1. Nanoporöser Sensor, bestehend aus einer Silikat-Sol-Gel-Matrix, die eine Reagenzzusammensetzung enthält, wobei die Reagenzzusammensetzung eine Mischung aus 4-(Dimethylamino)-cinnamaldehyd und Polystyrolsulfonsäure umfasst.

2. Nanoporöser Sensor gemäß Anspruch 1, **gekennzeichnet durch** eine spezifische Adsorptionsfläche von 700 bis 2500 $m^2.g^{-1}$, vorzugsweise von 800 bis 2000 $m^2.g^{-1}$.

3. Nanoporöser Sensor nach Anspruch 1 oder 2, **gekennzeichnet durch** ein Porenvolumen von 0,1 bis 0,9 $cm^3.g^{-1}$, bevorzugt von 0,2 bis 0,8 $cm^3.g^{-1}$, noch bevorzugter von 0,2 bis 0,6 $cm^3.g^{-1}$.

4. Nanoporöser Sensor gemäß einem der Ansprüche 1 bis 3, **gekennzeichnet durch** einen Anteil an Mikroporen von mehr als 75 %, bevorzugt mehr als 80 %, noch bevorzugter von 85 % bis 95 %, wobei der Rest bis 100 % dem Anteil an Mesoporen entspricht.

5. Verfahren zur Herstellung eines nanoporösen Sensors gemäß einem der Ansprüche 1 bis 4, wobei das Verfahren die folgenden Schritte umfasst:

   (a) Synthese eines Sols aus einem Organosilylvorläufer, wobei die Synthese in einem Lösungsmittel durchgeführt wird, das Wasser umfasst, in Gegenwart von 4-(Dimethylamino)-cinnamaldehyd und Polystyrolsulfonsäure;
   (b) Formen des in Schritt (a) erhaltenen Sols, gefolgt von dessen Gelierung, um ein Gel zu erhalten;
   (c) Trocknen des in Schritt (b) erhaltenen Gels, gefolgt von dessen Entformen, um einen nanoporösen Sensor zu erhalten.

6. Verfahren gemäß Anspruch 5, **gekennzeichnet dadurch, dass** der Organosilylvorläufer aus Tetramethoxysilan, Methyltrimethoxysilan, Ethyltrimethoxysilan, 3-Aminopropyltriethoxysilan, 3-Aminopropyltrimethoxysilan, (3-(Methylamino)propyl)trimethoxysilan, 3-Carboxypropyltriethoxysilan, 3-Carboxypropyltrimethoxysilan, Tetraethoxysilan und deren Mischungen ausgewählt ist.

7. Verfahren zum Nachweis mindestens einer Aminverbindung in einer zu analysierenden Gasprobe, wobei die Verbindung aus Hydrazin, Ethanolamin, Ammoniak und Morpholin ausgewählt ist, unter Verwendung eines nanoporösen Sensors gemäß einem der Ansprüche 1 bis 4, wobei das Verfahren die folgenden Schritte umfasst:

   (a) Inkontaktbringen der zu analysierenden Gasprobe mit dem nanoporösen Sensor,
   (b) Nachweis der Aminverbindung(en) in der zu analysierenden Gasprobe auf dem nanoporösen Sensor.

8. Verfahren gemäß Anspruch 7, wobei Schritt (b) zusätzlich die Quantifizierung der Aminverbindung(en) in der zu analysierenden Gasprobe auf dem nanoporösen Sensor umfasst.

9. Verwendung eines nanoporösen Sensors gemäß einem der Ansprüche 1 bis 4 zum Nachweis oder zur Quantifizierung oder zum Nachweis und zur Quantifizierung mindestens einer Aminverbindung, wobei die Verbindung aus Hydrazin, Ethanolamin, Ammoniak und Morpholin ausgewählt ist.

10. Vorrichtung (1) zum Nachweis mindestens einer Aminverbindung in einer zu analysierenden Gasprobe, wobei die Verbindung aus Hydrazin, Ethanolamin, Ammoniak und Morpholin ausgewählt ist, wobei die Vorrichtung umfasst:

    - eine Zelle (11), die einen nanoporösen Sensor gemäß einem der Ansprüche 1 bis 4 enthält und umfasst:

       - einen Gaseinlass (111);
       - einen Gasauslass (112);
       - einen optischen Eingang (113);
       - einen optischen Ausgang (114).

**Claims**

1. Nanoporous sensor composed of a silicate sol-gel matrix containing a reagents composition, said reagents composition comprising a mixture of 4-(dimethylamino)cinnamaldehyde and polystyrenesulfonic acid.

2. Nanoporous sensor according to Claim 1, **characterized in that** it has a specific surface area for adsorption of 700 to 2500 $m^2.g^{-1}$, preferably of 800 to 2000 $m^2.g^{-1}$.

3. Nanoporous sensor according to Claim 1 or 2, **characterized in that** it has a pore volume of 0.1 to 0.9 $cm^3.g^{-1}$, preferably of 0.2 to 0.8 $cm^3.g^{-1}$, even more preferentially of 0.2 to 0.6 $cm^3.g^{-1}$.

4. Nanoporous sensor according to one of Claims 1 to 3, **characterized in that** it has a proportion of micropores of greater than 75%, preferably greater than 80%, more preferentially ranging from 85% to 95%, the remainder to 100% corresponding to the proportion of mesopores.

5. Process for preparing a nanoporous sensor according to one of Claims 1 to 4, said process comprising the following steps:

   a. synthesis of a sol from an organosilyl precursor, the synthesis being performed in a solvent, said solvent comprising water, in the presence of 4-(dimethylamino)cinnamaldehyde and polystyrenesulfonic acid;
   b. molding of the sol obtained in step a), followed by gelation to obtain a gel;
   c. drying of the gel obtained in step b), followed by its demolding to obtain a nanoporous sensor.

6. Process according to Claim 5, **characterized in that** the organosilyl precursor is chosen from tetramethoxysilane, methyltrimethoxysilane, ethyltrimethoxysilane, 3-aminopropyltriethoxysilane, 3-aminopropyltrimethoxysilane, (3-(methylamino)propyl)trimethoxysilane, 3-carboxypropyltriethoxysilane, 3-carboxypropyltrimethoxysilane, tetraethoxysilane and mixtures thereof.

7. Process for detecting in a gas sample to be analyzed at least one amine-type compound, said compound being chosen from hydrazine, ethanolamine, ammonia and morpholine, using a nanoporous sensor according to one of Claims 1 to 4, said process comprising the steps of:

   a. placing said gas sample to be analyzed in contact with said nanoporous sensor,
   b. detecting on said nanoporous sensor said amine-type compound(s) in the gas sample to be analyzed.

8. Process according to Claim 7, in which step b) also comprises quantification on said nanoporous sensor of said amine-type compound(s) in the gas sample to be analyzed.

9. Use of a nanoporous sensor according to one of Claims 1 to 4, for the detection or quantification or the detection and quantification of at least one amine-type compound, said compound being chosen from hydrazine, ethanolamine, ammonia and morpholine.

10. Device (1) for detecting in a gas sample to be analyzed at least one amine-type compound, said compound being chosen from hydrazine, ethanolamine, ammonia and morpholine, said device comprising:

   - a cell (11) enclosing a nanoporous sensor according to one of Claims 1 to 4 and comprising:

      - a gas inlet (111);
      - a gas outlet (112);
      - an optical input (113);
      - an optical output (114).

[Fig. 1]

[Fig. 2]

[Fig. 3]

[Fig. 4]

[Fig. 5]

EP 4 553 496 B1

[Fig. 6]

[Fig. 7]

[Fig. 8]

22

[Fig. 9]

[Fig. 10]

EP 4 553 496 B1

[Fig. 11]

[Fig. 12]

[Fig. 13]

24

[Fig. 14]

[Fig. 15]

[Fig. 16]

[Fig. 17]

## RÉFÉRENCES CITÉES DANS LA DESCRIPTION

**Documents brevets cités dans la description**

- US 005719061 A, Rose-Pehrsson
- US 2012295363 A1

**Littérature non-brevet citée dans la description**

- IUPAC. Compendium of Chemical Terminology. Blackwell Scientific Publications, 1997
- *CHEMICAL ABSTRACTS*, 681-84-5
- *CHEMICAL ABSTRACTS*, 6203-18-5
- *CHEMICAL ABSTRACTS*, 28210-41-5
- *CHEMICAL ABSTRACTS*, 6192-52-5
- **OSHA**. *Sampling and Analytical methods*, 1997, https://www.osha.gov/dts/sltc/methods/organic/org108/org108.html
- **B. J. MENEGHELLI**. A review of hydrazine sensors: The state of the art. ASRC Aerospace Corp., 2004
- **K. P. BRENNER** ; **S. L. ROSE-PEHRSS**. *Performance Evaluation of a Colorimetric Hydrazine Dosimeter*, 1994